# EUROPEAN PATENT SPECIFICATION

(11) **EP 3 071 255 B1**
(45) Date of publication and mention of the grant of the patent: **02.09.2020**
(21) Application number: 14862518.9
(22) Date of filing: 18.11.2014
(51) Int. Cl.: A61M 5/168, A61M 25/16, A61M 25/18, A61M 39/22, A61M 39/26

(54) **MEDICAL LUER CONNECTOR**
MEDIZINISCHER LUER-ANSCHLUSS
CONNECTEUR LUER MÉDICAL

(30) Priority: 18.11.2013 US 201361905775 P
(43) Date of publication of application: 28.09.2016
(73) Proprietor: HALKEY-ROBERTS CORPORATION, St. Petersburg, FL 33716-4103 (US)
(72) Inventor: BONALDO, Jean, Upland, CA 91786 (US)
(74) Representative: Franks & Co Limited
(86) International application number: PCT/US2014/066141
(87) International publication number: WO 2015/074044

(56) References cited:
- EP-B1- 1 189 653
- WO-A1-01/93936
- US-A- 4 220 151
- US-A- 5 947 954
- US-A- 5 947 954
- US-A1- 2003 116 162
- US-A1- 2003 116 162
- US-A1- 2006 157 984
- US-A1- 2006 157 984
- US-B2- 7 329 249
- US-B2- 7 329 249

## Description

### CROSS-REFERENCE TO RELATED INVENTIONS

This application claims the benefit of pending provisional application number 61/905,774, filed November 18, : 2013.

### BACKGROUND OF THE INVENTION

### Field of the Invention

This invention relates to luer connectors.

### Description of the Background Art

Presently, luer connectors are standardly used to connect medical devices involved in transferring medical fluids, and can be found for example on syringes, catheters, and connectors for IV tubing. Luer connectors consist of interlocking male (outer) and female (inner) portions which are slightly tapered so as to connect via a pressure or interference fit in a fluid- tight manner. When the male and female portions are joined with a threaded connection, the connection is referred to as a luer lock, which opens and closes by twisting. A non-threaded luer connection is generally referred to as a luer plug or luer slip.

Male and female luer connector portions cooperate to communicatively couple separate devices or pieces of medical tubing and form a single flow pathway. For example, one length of tubing can be attached to a male luer connector and another length of tubing can be attached to a female luer connector. The separate lengths of tubing can then be placed into fluid communication by coupling the male luer connector portion and the female luer connector portion.

In some circumstances, luer connectors may be connected to and disconnected from flowlines on at least a daily basis. Repeated usage of a luer connector, however, may cause the connector to leak or become contaminated with particulate material, such as particles that detach from the septum of the connector and/or cotton fibers from swabs used to clean the connectors. Connectors must remain leak free and reliably avoid introduction of contaminants.

A variety of luer connections are known. For example, e.g. US 2003/116162 Madsen discloses a snap fit medical connector for a respiratory apparatus comprising first and second interlocking parts, having an optional snap fit member present on at least one of the first and second connection members. US 2006/157984 Rome discloses a further type of locking medical luer fitting. U.S. Patent Nos. 5,273,533 and 5,306,243 to Bonaldo, for example, disclose a medical connector having an elastomeric element in the form of a septum or fluid barrier disposed in a two part plastic housing. The septum is pierced by a pointed cannula in the connector when making the connection to the fluid flowline. U.S. 7,329,249 Bonaldo discloses a medical fluid flowline connector comprising axially aligned relatively rotatable male and female luer parts; the female luer part being provided with an elastomeric stopper having a slit to ensure deformation and opening of the slot in response to the male luer. U.S. Patent No. 5,947,954 to Bonaldo discloses a needleless connector which includes attached relatively rotatable male and female luer connector parts with an eccentrically positioned flow passageway at the inner end of the female luer connector. Further needleless luer connectors include that of U.S. Patent No. 7,118,560 Bonaldo and WO 01/93936 of Creative Plastic Technology LLC.

Therefore, it is an object of this invention to provide an improvement which overcomes the aforementioned inadequacies of the prior art devices and provides an improvement which is a significant contribution to the advancement of the medical luer connector art.

The foregoing has outlined some of the pertinent objects of the invention. These objects should be construed to be merely illustrative of some of the more prominent features and applications of the intended invention. Many other beneficial results can be attained by applying the disclosed invention in a different manner or modifying the invention within the scope of the disclosure. Accordingly, other objects and a fuller understanding of the invention may be had by referring to the summary of the invention and the detailed description of the preferred embodiment in addition to the scope of the invention defined by the claims taken in conjunction with the accompanying drawings.

### SUMMARY OF THE INVENTION

There remains a need to provide a further improved medical connector which includes a female luer end having an elastomeric stopper which still further reduces the likelihood of contaminant entry to the fluid flow path of the connector.

The foregoing has outlined rather broadly the more pertinent and important features of the present invention in order that the detailed description of the invention that follows may be better understood so that the present contribution to the art can be more fully appreciated. Additional features of the invention will be described hereinafter which form the subject of the claims of the invention. It should be appreciated by those skilled in the art that the conception and the specific embodiment disclosed may be readily utilized as a basis for modifying or designing other structures for carrying out the same purposes of the present invention. It should also be realized by those skilled in the art that such equivalent constructions do not depart from the spirit and scope of the invention as set forth in the appended claims.

### BRIEF DESCRIPTION OF THE DRAWINGS

For a fuller understanding of the nature and objects of the invention, reference should be had to the following detailed description taken in connection with the accompanying drawings in which:
Figure 1 is a top plan view of the present medical luer connector assembly.
Figure 2 is a side elevation view of the present medical luer connector assembly.
Figure 3 is a sectional view of the present medical luer connector assembly along line 2-2 of Figure 2.
Figure 4 is a perspective view of the male luer component of the present luer connector.
Figure 5 is a rear elevation view of the male luer component of the present luer connector.
Figure 6 is side elevation view of the male luer component of the present luer connector.
Figure 7 is a sectional view of the male luer component of the present luer connector along line 6-6 of Figure 6.
Figure 8 is a top plan view of the male luer component of the present luer connector.
Figure 9 is a sectional view of the male luer component of the present luer connector along line 8-8 of Figure 8.
Figure 10 is a perspective view of a contamination cap for the male luer component of the present luer connector.
Figure 11 is a perspective view of the female luer component of the present luer connector.
Figure 12 is a side elevation view of the female luer component of the present luer connector.
Figure 13 is a sectional view of the female luer component of the present luer connector along line 12-12 of Figure 12.
Figure 14 is an elevation view of the distal end of the female luer component of the present luer connector.
Figure 15 is a perspective view of the female luer assembly of the present luer connector.
Figure 16 is a front elevation view of the female luer assembly of the present luer connector.
Figure 17 is a rear elevation view of the female luer assembly of the present luer connector.
Figure 18 is a side elevation view of the female luer assembly of the present luer connector.
Figure 19 is a sectional view of the female luer component of the present luer connector along line 18-18 of Figure 18.
Figure 20 is a sectional view of the female luer component of the present luer connector along line 19-19 of Figure 19.
Figure 21 is a perspective view of the channel insert of the female luer assembly of the present luer connector.
Figure 22 is a side elevation view of the channel insert of the female luer assembly of the present luer connector.
Figure 23 is a sectional view of the channel insert of the female luer component of the present luer connector along line 22-22 of Figure 22.
Figure 24 is a rear elevation view of the channel insert of the female luer assembly of the present luer connector.
Figure 25 is a front elevation view of the channel insert of the female luer assembly of the present luer connector.
Figure 26 is a side elevation view of the elastomeric stopper of the female luer assembly of the present luer connector.
Figure 27 is a sectional view of the elastomeric stopper of the female luer assembly of the present luer connector along line 26-26 of Figure 26.
Figure 28 is a perspective view of the seal component of the present luer connector.
Figure 29 is a rear elevation view of the seal of the present luer connector.
Figure 30 is a side elevation view of the seal of the present luer connector.
Figure 31 is a front elevation view of the seal of the present luer connector.
Figure 32 is a sectional view of the seal of the present luer connector along line 31- 31 of Figure 31.

Similar reference characters refer to similar parts throughout the several views of the drawings.

### DETAILED DESCRIPTION OF THE PREFERRED EMBODIMENT

### Definitions

As used herein, the following terms and variations thereof have the meanings given below, unless a different meaning is clearly intended by the context in which such term is used.

The terms "a," "an," and "the" and similar referents used herein are to be construed to cover both the singular and the plural unless their usage in context indicates otherwise.

"Annular" refers a component or portion thereof forming a ring, and is inclusive of a partial or complete ring-shaped structure, for example, a tubular structure, a "c-shape," a "u- shape," or an "o-shape."

"Cam and groove" fitting or coupling refers to a connection between two components in which a projection or "cam" on one component fits into a "cam surface" or groove in another component, in particular in order to effect a seal against a seal or gasket.

"Connected" and "coupled" refer to components that are either directly connected or coupled to another element or which are separated by intervening elements. When an element is referred to as being "directly connected" or "directly coupled" to another element, there are no intervening elements present.

"Comprise" and variations of the term, such as "comprising" and "comprises," are not intended to exclude other additives, components, integers or steps. "Elastic" refers to a material or component which can be bent or otherwise deformed elastically by a predetermined force but which will return to its original shape when the applied force is removed.

"Elastic" refers to a material or component which can be bent or otherwise deformed elastically by a predetermined force but which will return to its original shape when the applied force is removed.

"Elastomer" and "elastomeric" refer to an elastic polymer material such as natural rubber, silicone rubber, and the like.

"Elongated" refers to a configuration or shape having a length which is longer than its width.

"Flange" refers to a projecting rim, collar, or ring on a structural element of a component of the present device which supports or provides a place of attachment for other components.

"Fluid conduit" and "conduit" refer to a structure for receiving and/or transferring fluids, for example medical tubing, a catheter, a drain tube, an access port, or a needle.

"Inward" and "inwardly" mean in a direction toward the longitudinal axis or center of the present device or of a component part.

"Longitudinal" refers to a direction or orientation aligned with the length (longer portion) of the present device or a component thereof.

"Male luer member" refers to a component of the present device comprising a projecting conduit having an open distal (outer) end and which tapers toward its distal end in order to allow connection to another fluid conduit.

"Outward" and "outwardly" mean in a direction away from the longitudinal axis or center of the present device or of a component part.

"Rim" refers to an outer edge of a component of the present device.

Relative terms, such as "lower", "bottom", "below", "upper", "top" or "above," may be used herein to describe one element's relationship to another element as illustrated in the Figures. Such relative terms are intended to encompass different orientations of the device in addition to the orientation depicted in the Figures.

### Luer Connector

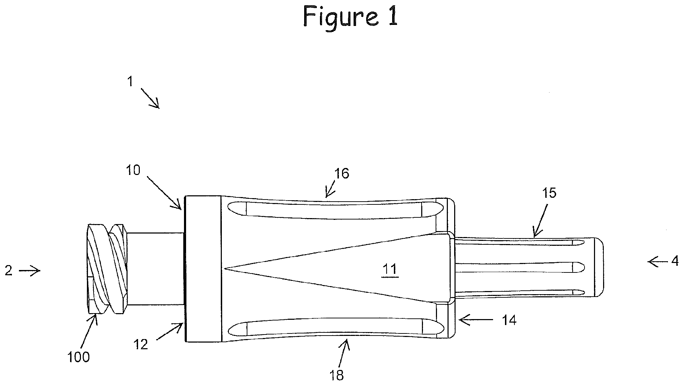

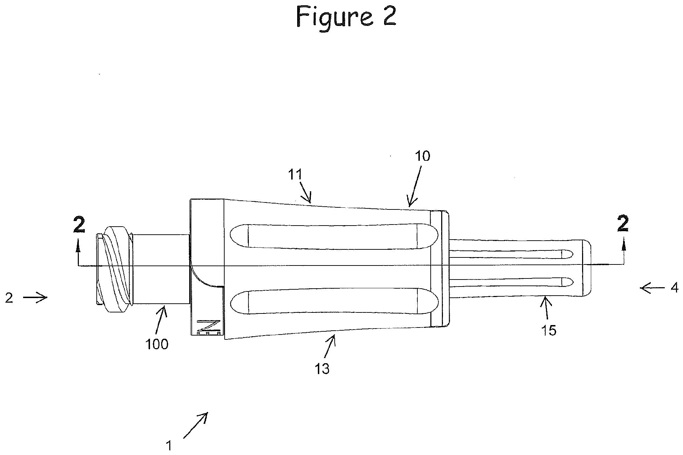

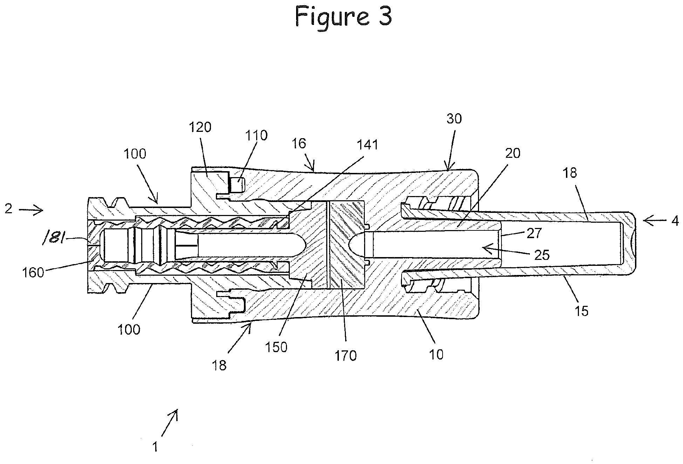

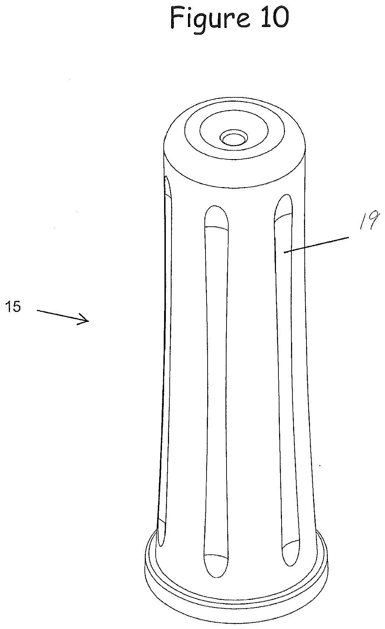

Figures 1 through 3 illustrate the present connector assembly 1. The assembly comprises a female luer body 100 in a proximal portion 2 of the assembly 1 and a male luer body 10 in a distal portion 4. The distal portion 4 optionally includes a cap 15 for covering a male luer member 20. The cap 15 includes a tapered inner surface 18 in order to mate with the exterior surface of the male luer member 20, and in heat-sterilized embodiments also preferably includes vents 16 (see Figure 10). As shown in Figure 3, the assembly 1 further includes a seal 170 and a channel insert 150, which together with the male luer body 10 forms a flow channel for fluids being transferred through the connector 1.

### Male Luer Body

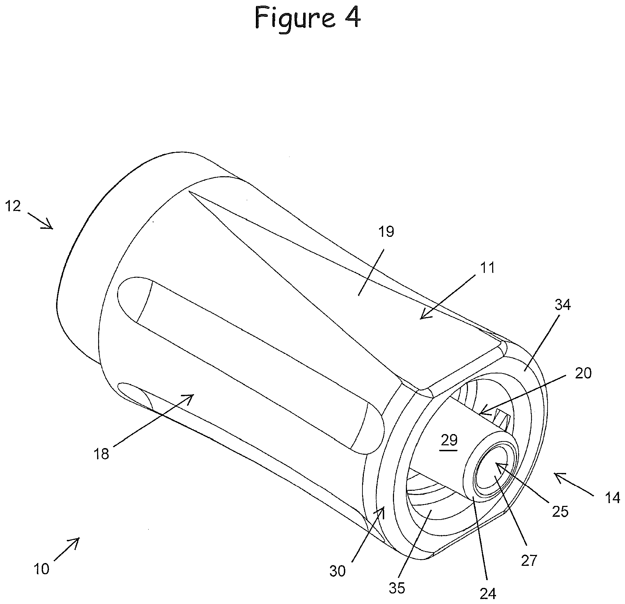

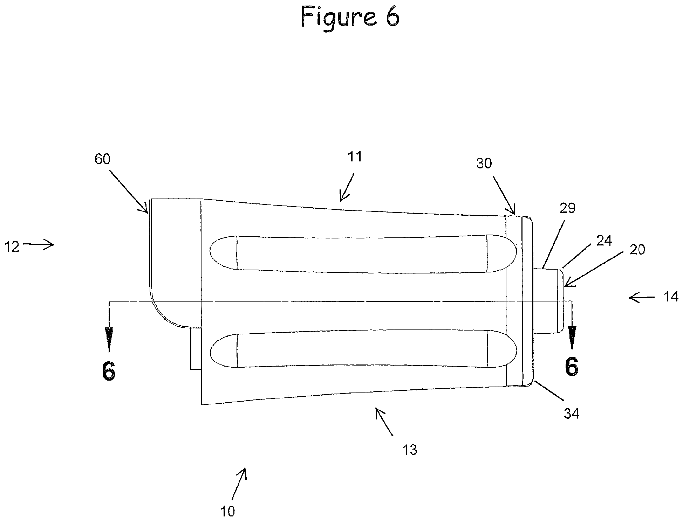

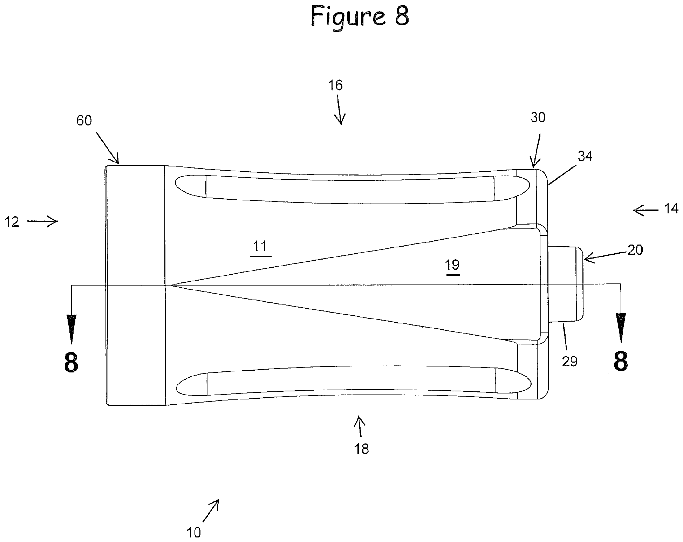

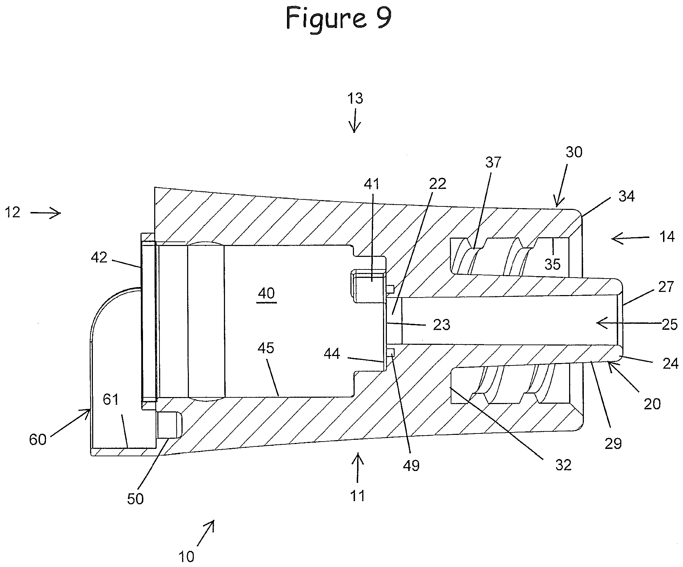

The male luer body 10 of the present connector assembly 1 is shown in Figures 4- 9. The male luer body 10 includes a proximal end 12, a distal end 14, an upper side 11, a lower side 13, and two lateral sides 16 and 18. The upper side 11 and/or lower side 13 preferably include a relatively flat, planar portion 19 in order to allow better gripping of the assembly 1 when being handled by a user, particularly when it is necessary to turn or twist the assembly 1 , and to deter rolling of the male luer body 10 when placed on a flat surface. In the illustrated embodiments, the planar portion 19 is triangular in shape, i.e. such that one longitudinal end is visually distinguishable from the other longitudinal end, in order to facilitate the orientation of the connector assembly 1 , though other non- symmetrical shapes can also be used. At the distal end 14, the male luer body 10 includes a longitudinally projecting male luer member 20 having a proximal end 22 and a distal end 24. The male luer member 20 comprises an inner lumen 25. The proximal end 22 comprises a proximal opening 23 at one end of the lumen 25, and the distal end 24 comprises a distal opening 27 at the other end, which together form a portion of the fluid flow path through the present connector assembly 1. The diameter of the outer surface 29 of the male luer member 20 decreases from the proximal end 22 to the distal end 24 in order to form a taper.

The distal end 24 of the male luer member 20 preferably extends longitudinally beyond the distal end 34 of an annular, preferably circumferential flange or collar 30 which surrounds the male luer member 20. Between the distal end 34 and the proximal and 32 of the collar 30, the inner surface 35 is preferably provided with grooves 37 to engage the threads of another fluid transfer device to which the present connector assembly 1 can be connected to form a fluid flow path.

At the distal end 24 of the male luer member 20, the male luer body 10 comprises a receptacle 40 for receiving the female luer assembly 105 having a proximal end 42, a distal end 44, and an inner surface 45. The distal end 44 includes seal pegs 41 (three of them in the illustrated embodiment) and an O-ring groove 49 for retaining a seal 170 in the receptacle 40.

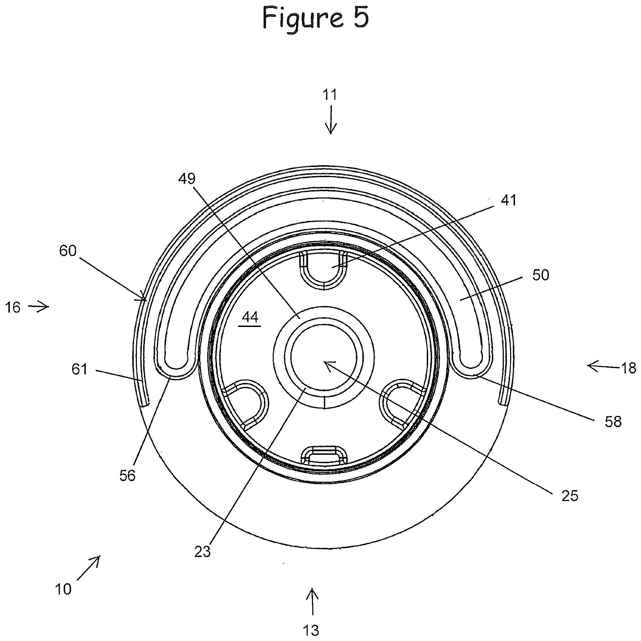

The proximal end 12 of the male luer body 10 further includes an annular, semicircular cam groove 50. As best seen in Figure 5, the cam groove 50 extends around a portion of the circumference of the proximal end 12 of the male luer body 10, and is shaped to receive and cooperate with a cam post 110 on the female luer body 100. The proximal end 12 of the male luer body 10 also preferably comprises a semicircular, annular guard or shield 60 which extends around a portion of the periphery of the proximal end 12 and has an inner surface 61. The guard 60 extends longitudinally and proximally of the proximal end 42 of the receptacle 40, in order to help prevent unintentional rotation of the female luer body 100, which might accidentally disconnect it from the male luer body 10. This is a particular issue when users are wearing protective gloves, which decrease the dexterity of the user. The guard also helps to prevent externally spilled fluid from getting in between the female luer body 100 and the male luer body 10.

### Female Luer Assembly

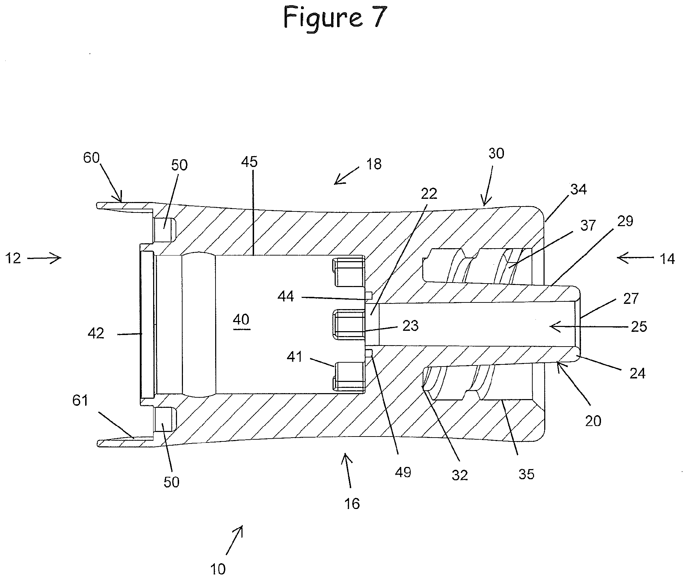

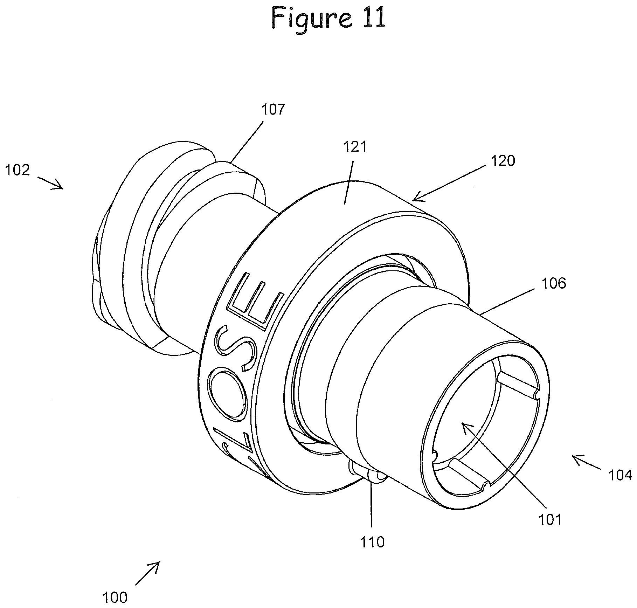

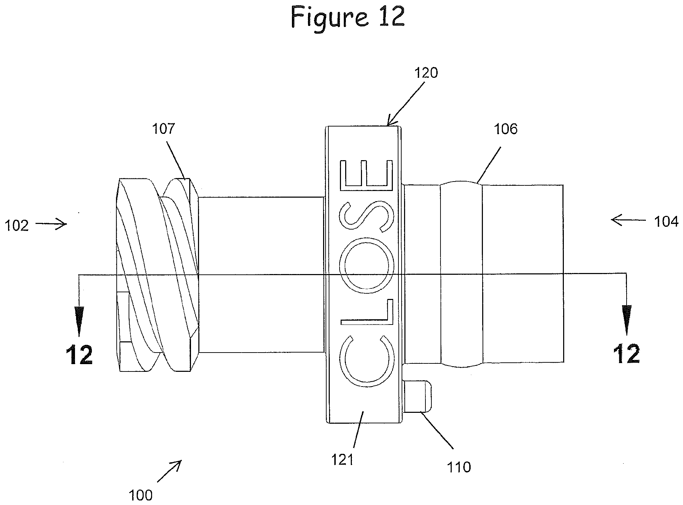

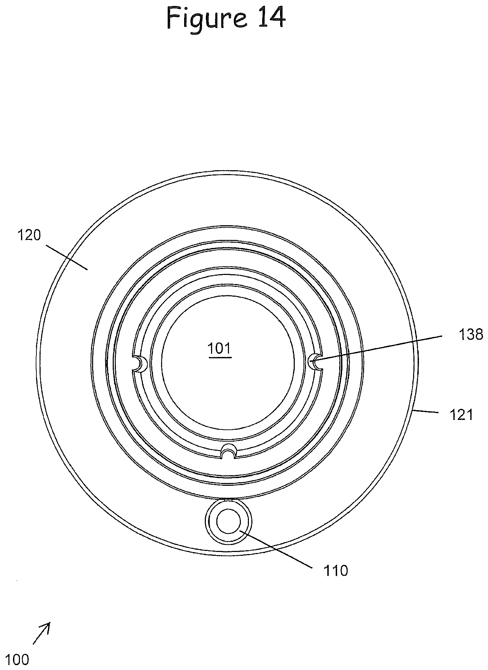

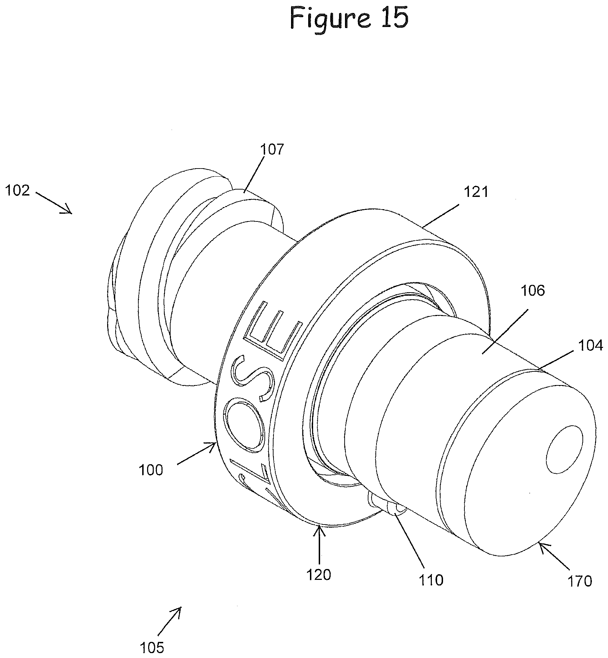

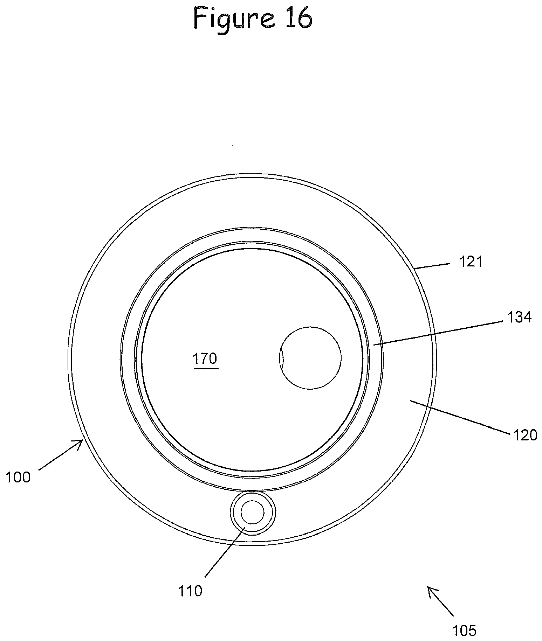

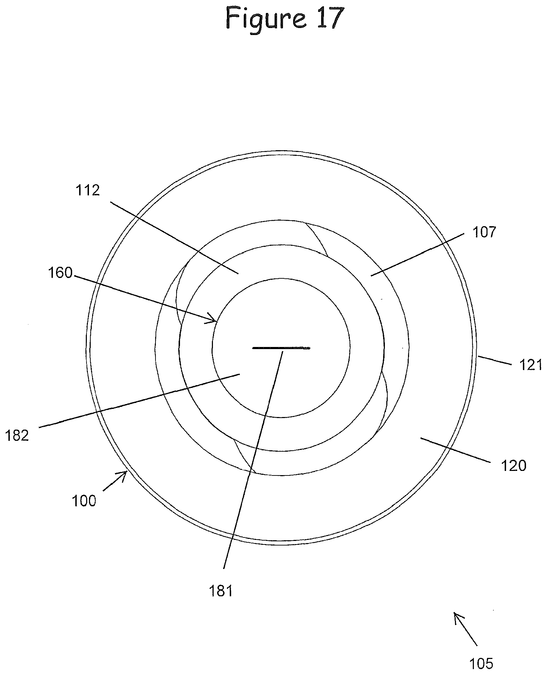

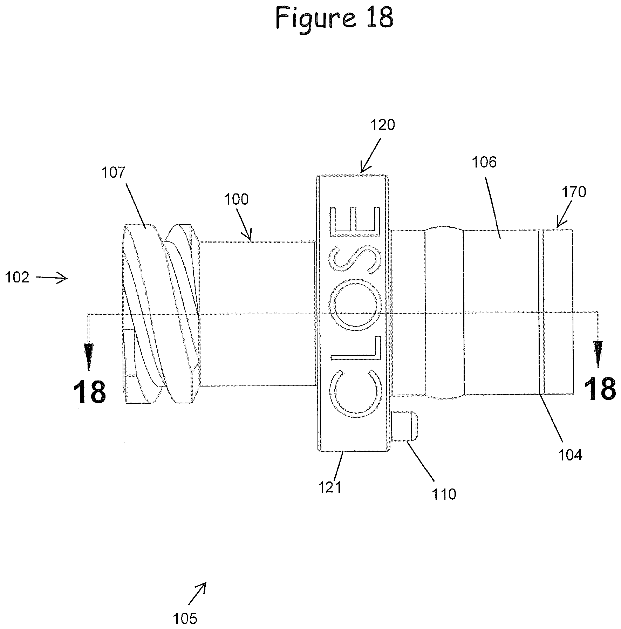

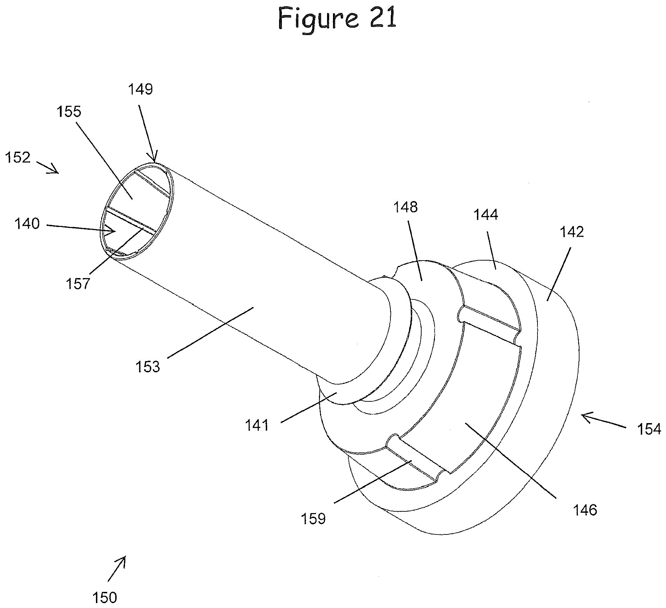

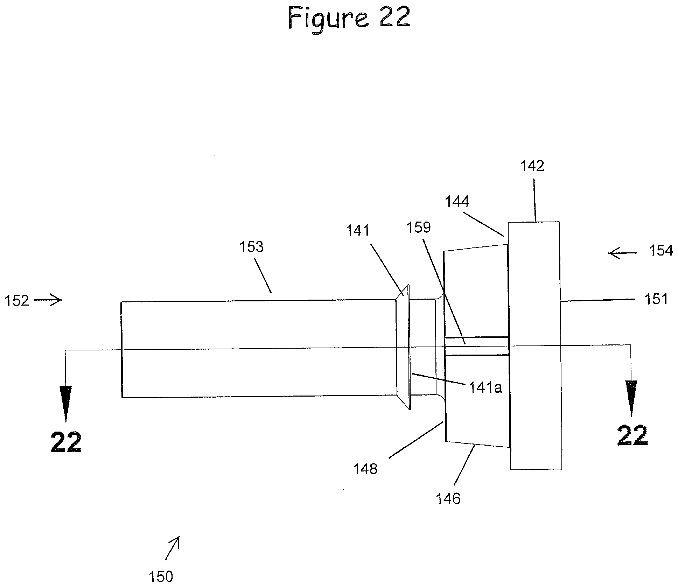

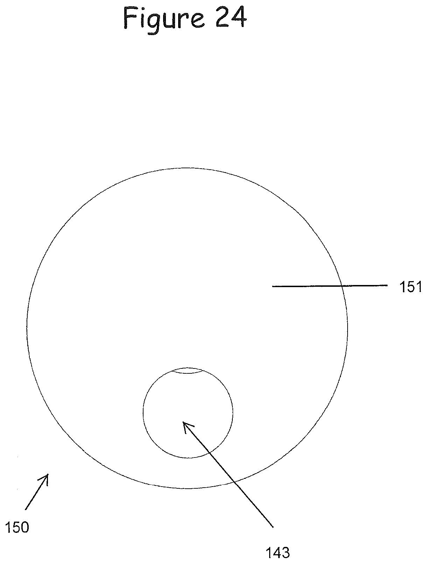

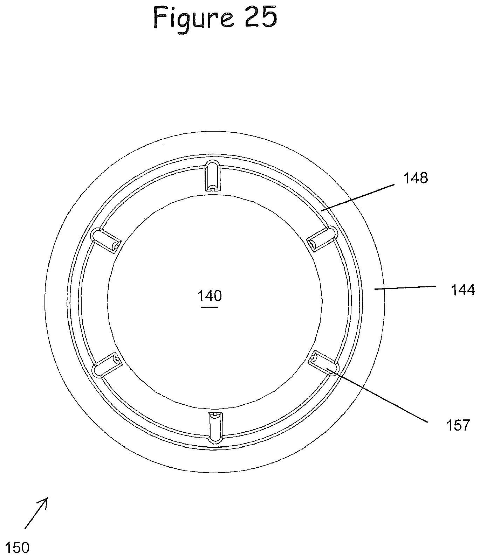

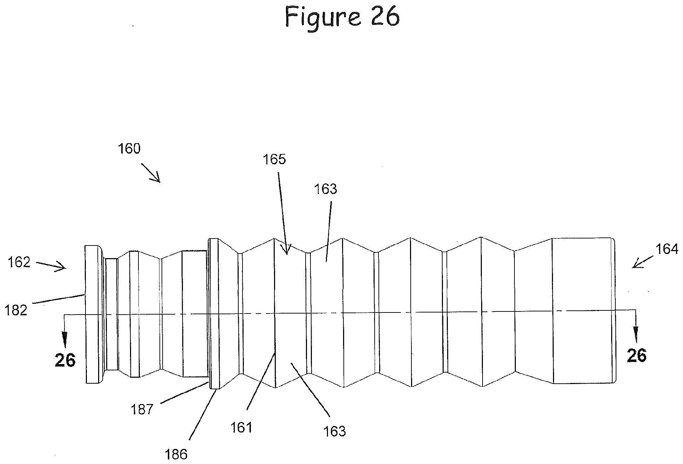

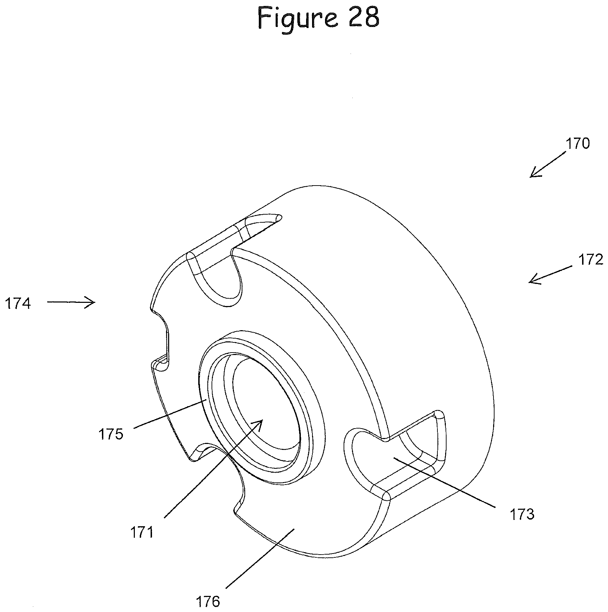

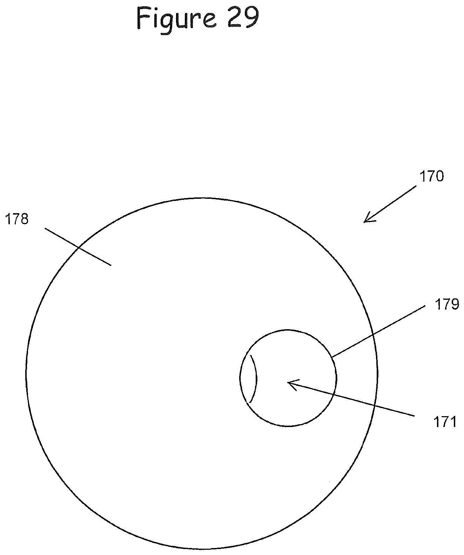

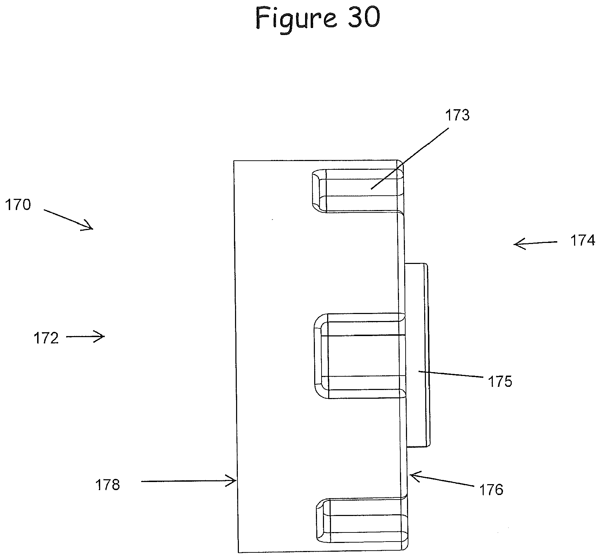

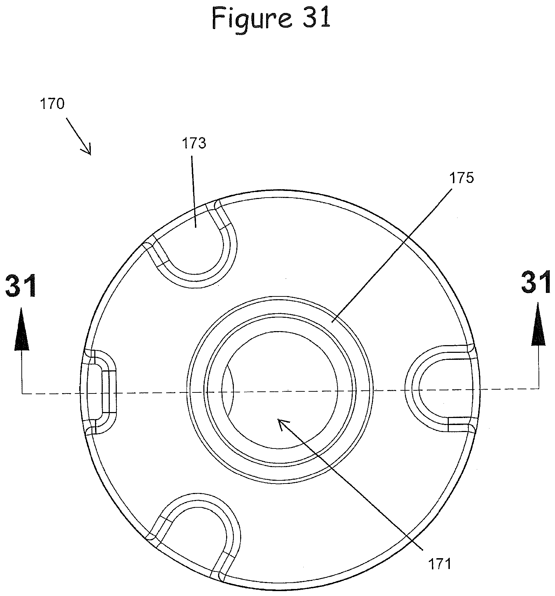

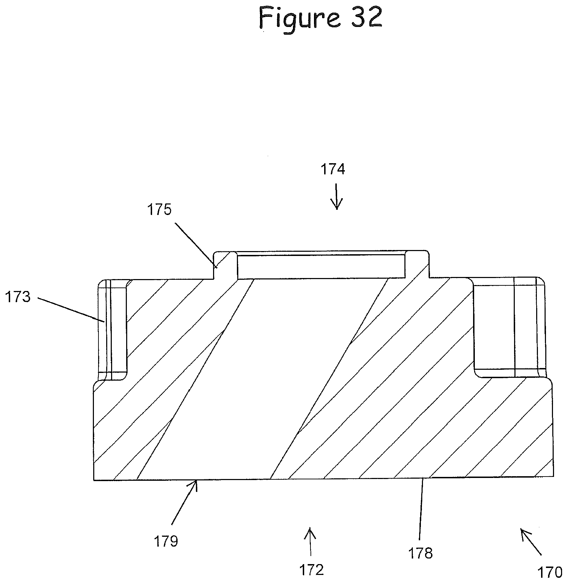

The receptacle 40 of the male luer body 10 (see Figure 7) is adapted to receive a female luer assembly 105. The female luer assembly 105 (Figs. 15-20) includes a female luer body (Figs. 11-14), a seal 170 (Figs. 28-32) at the distal end 44 of the receptacle 40, a channel insert 150 (Figs. 21-25) adjacent the seal 170, and a stopper 160 (Figs. 26-27) at the proximal end 42 of the receptacle 40.

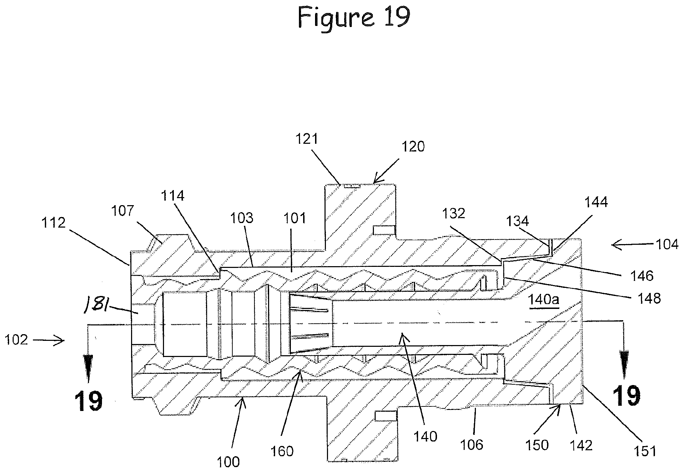

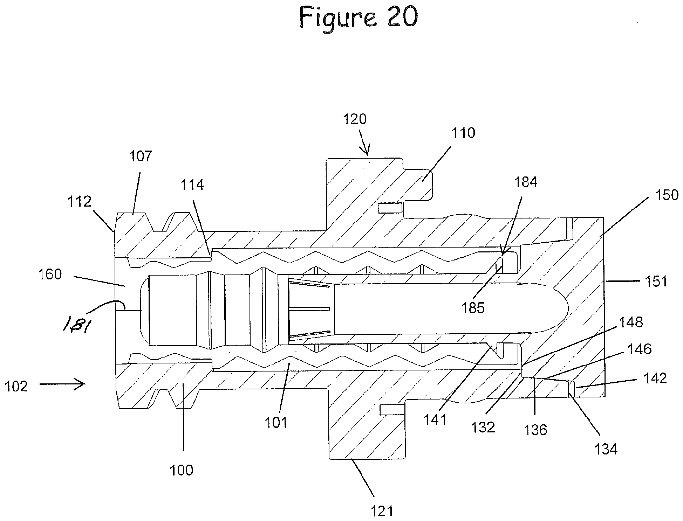

As best seen in Figures 11-14, the female luer body 100 comprises a proximal end 102, a distal end 104, and an interior 101 for retaining the channel insert 150 and stopper 160 (as shown in Figures 19 and 20). Proximal end 102 preferably includes threads 107 for mating with the grooves of another fluid transfer device. The distal end 104 of the female luer body 100 is adapted to fit within the receptacle 40 of the male luer body 10. When the female luer body 100 is retained within the male luer body, the outer surface 106 of the proximal end 104 is adjacent to, and preferably is in contact with, the inner surface 45 of the receptacle 40 of the male luer body 10 (Figure 3).

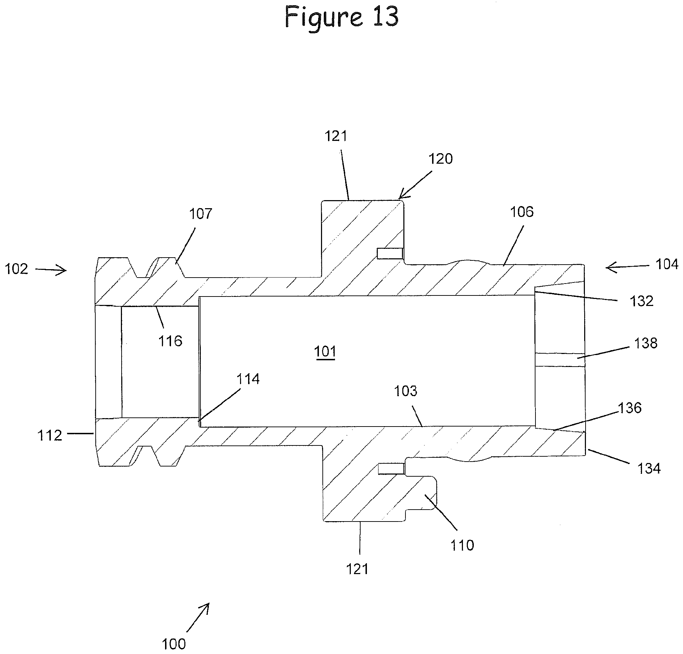

As best seen in Figure 13, the interior 101 of the female luer body 100 comprises an inner surface 103 for retaining components of the female luer assembly 105. At proximal end 102, the interior 101 includes an inwardly extending annular wall or surface 114 which extends inwardly from a proximal end of the inner surface 103. This inwardly extending wall 114 helps to retain the elastomeric stopper 160 (as described further below). A proximal inner surface 116 extends proximally from the inwardly extending wall 114 toward the proximal end 102, ending at exterior proximal surface 112. At a distal end of the inner surface 103, and outwardly extending annular wall or surface 132 extends outwardly, in order to help retain the channel insert 150. A distal inner surface 136 frusto conically extends distally from the outwardly extending surface 132, ending as an exterior distal surface 134.

The female luer body 100 preferably includes a cam post 110 which can be retained by the cam groove 50 of the male luer body 10. The cam post 110 allows the female luer body 100 to be rotated until the cam post 110 reaches one of the lateral ends 56 or 58 of the cam groove 50 (Figure 5), thereby preventing over-torquing of the female luer body 100. The cam post 110 and cam groove 50 also cooperate to prevent separation of the male luer body 10 and the female luer body 100 under conditions of high pressure. The cam post 110 extends longitudinally toward the distal end 104 of the female luer body 100 from an annular, preferably circumferential flange 120 extending outwardly from a medial portion of the female luer body 100. The flange 120 facilitates rotation of the female luer body 100 when attaching it to or detaching it from the male luer body 10 (at the distal end 104) and/or another fluid transfer device (at the proximal end 102). The outer surface 121 of the flange 120 preferably abuts the inner surface 61 of the guard 60 of the male luer body 10 when the male luer body 10 and female luer body 100 are connected.

As best seen in Figures 19 and 20, the female luer body 100 engages and retains a channel insert 150, which is shown in Figures 21-25. A channel insert 150 generally comprises a proximal end 152, a distal end 154, and an interior channel 140 extending between the proximal end 152 and the distal end 154. The proximal end 152 of the channel insert 150 generally comprises a preferably tubular conduit 153 having an opening 149 at its proximal end. At or adjacent the opening 149, the interior surface 155 of the channel 140 preferably comprises generally longitudinally extending grooves 157. The distal end 154 of the channel insert 150 includes an outwardly extending medial surface or wall 148, which extends outwardly from the outer surface of the conduit 153. The outwardly extending medial wall 148 meets an annular medial surface or wall 146, which extends generally frustroconically (longitudinally and distally) to an outwardly extending distal wall 144. At the outer perimeter of the outwardly extending distal wall 144, an annular distal surface or wall 142 extends outwardly to the distal surface 151 of the channel insert 150. An annular barb-shaped flange or ring 141 extends outwardly from the tubular conduit 153 between the outwardly extending medial wall 148 and the proximal end 152 of the channel insert 150.

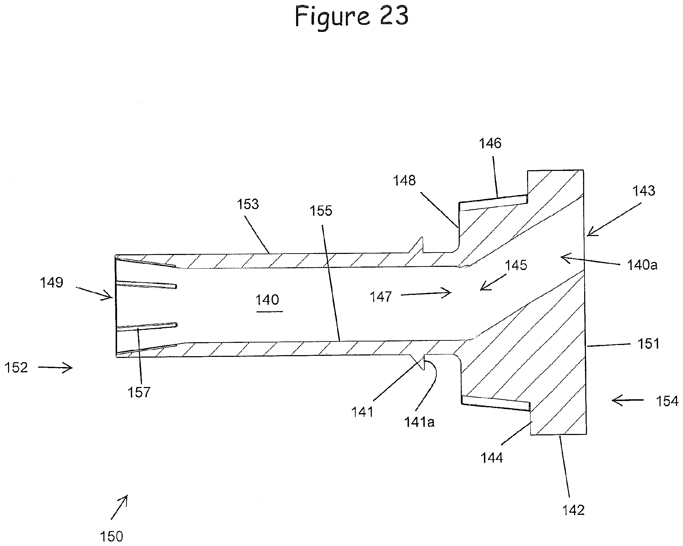

As best seen in Figure 23, the channel 140 extends from the opening 149 distally to a distal channel opening 147. The distal channel opening 147 meets and/or is integrally formed with a proximal side channel opening 145 for a side channel 140a. The distal channel 140a meets the interior channel 140 at an angle, in order to position the channel distal opening 143 away from the center of the distal surface 151 of the channel insert 150. The use of an offset opening 143 ensures that this opening aligns with the corresponding opening on the seal 170, thereby allowing fluid flow through the connector 1, only when the female luer body 100 and the channel insert 150 which it retains are secured to the male luer body 10. When the female luer body 100 is not appropriately rotated and securely retained within the male luer body 10, the opening 143 of the channel insert 150 will not align, or not fully align, with the corresponding opening 179 on the seal 170 and thereby block or substantially hinder fluid flow through the device.

The distal end 104 of the female luer body 100 generally engages the channel insert 150 at the distal end 154 of the channel insert 150. As seen for example in Figure 19, the exterior distal surface 134 of female luer body 100 abuts distal wall 144 of the channel insert 150; distal inner surface 136 of the female luer body 100 abuts the medial wall 146 of the channel insert 150; and outwardly extending surface 132 of the female luer body 100 abuts medial wall 148 of the channel insert 150. In order to securely retain and orient the channel insert within the female luer body 100, a plurality of longitudinal grooves 159 are provided on the medial wall 146 (see Figs. 21-23). The grooves 159 are engaged by longitudinally extending projections or cams 138 on the distal inner surface 136 of the distal end of the female luer body 100 (see Figs. 11 and 13-14). The projections 138 prevent or resist rotational movement of the channel insert 150 when engaged by the grooves 159 of the channel insert 150.

A further advantage of the present channel insert 150 is that it prevents contact between the interior 101 of the female luer body 100 and fluids passing through the connector assembly 1. This eliminates the possibility of contamination of such fluids due to contact with the interior 101.

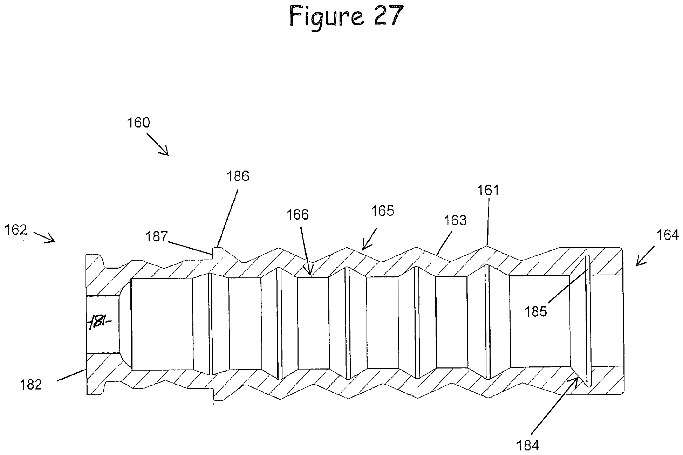

The female luer assembly 105 further comprises an axially (longitudinally) deformable elastomeric stopper 160, best seen in Figures 26 and 27. The stopper 160 generally comprises a hollow, longitudinal structure having bellows or accordion-like folds 161, with each fold being at the juncture of two opposed surfaces 163 on the exterior 165 of the stopper 160. The stopper is mounted or positioned around the channel insert 150, such that the interior surface 166 of the stopper 160 surrounds the exterior surface of the tubular conduit 153 of the channel insert (as seen in Figures 3, 19, and 20, for example). When force is exerted against the closed, proximal face 182 of the stopper 160 by a syringe or other fluid transfer device, the proximal face 182 is urged distally toward the proximal end 152 of the channel insert 150, and the opposed surfaces 163 of the accordion- like outer surface 165 are urged together, thereby collapsing the stopper 160 and reducing it in length. This allows a syringe, for example, to be inserted into the proximal end of the female luer body 100. When the proximal end 162 of the stopper 160 reaches the proximal 182 into the opening 149 of the channel 140 of the channel insert 150. The elastic material of the proximal face 182 will be urged into the longitudinally extending grooves 157 of the channel insert 150, which thereby grip the material of the stopper 160. The stopper 160 will resist twisting when gripped in this way, which is advantageous because twisting tends to weaken and/or tear the material, creating a risk of introducing an infection.

The stopper is made from an elastic, preferably elastomeric material such as silicone, so that once a fluid transfer device is removed from the female luer body, the proximal face 182 will move proximally to its original position. When not in use, the proximal face 182 of the stopper 160 is preferably flush with proximal wall 112 of the female luer body 100, so that both the proximal wall 112 and the proximal face 182 can be swabbed with a disinfectant. In one embodiment, the proximal face 182 is fully closed when the stopper 160 is not in use, and has a reversibly openable slit 181 in a central portion of the face 182. In this case the walls of the slit 181 preferably contact one another when the elastic material of the stopper 160 is relaxed (i.e. when the stopper 160 is not in use) in order to close the opening in the proximal end 162 of the stopper 160 and prevent contamination of the present device. The walls of the slit 181 can be urged open when a syringe or other fluid conduit presses against the proximal face 182 of the stopper 160.

Additional slits 181 can be included in the face 182, for example two slits intersecting to form an "x" can be used.

In order to better retain the elastic stopper 160 within the female luer body 100, the stopper 160 is provided with a retaining groove 184 having an inwardly extending annular wall 185, preferably at the distal end 164 of the stopper 160. The retaining groove 184 surrounds the annular flange 141 of the channel insert 150 (see Fig. 20) such that a distally facing, outwardly extending distal wall 141a is engaged by a proximally facing, inwardly extending surface 185 of the retaining groove 184 and restrains the lateral, and in particular proximal, movement of the stopper 160. In addition, the exterior 165 of the stopper 160 is preferably provided with an outwardly extending, annular projection 186 having a proximal face 187 which is adapted to engage the inwardly extending wall 114 of the interior 101 of the female luer body 100 (see Fig. 20.) This further restrains the proximal movement of the stopper 160.

The female luer body 100 and channel insert 150 are placed in fluid communication with the male luer member 20 of the male luer body 10 via a seal 170 (see Figs. 28-32), which is preferably formed from an elastomeric or other elastic material such as silicone. The seal 170 preferably has a generally cylindrical shape with a fluid flow passageway 171 extending from an off-center positioned proximal end 172 to an axially aligned distal end 174. The off-center opening 179 in the proximal face 178 aligns the fluid flow channel 171 with the channel distal opening 143 of the channel insert 150 in order to allow a flow of fluid through the connector assembly 1.

The seal 170 is preferably partially compressed between the distal surface 151 of the channel insert 150 and the wall at the distal end 44 of the male luer body 10. The seal 170 has at least one and preferably three axially extending grooves 173 on its annular surface which mate with axially extending seal pegs 41 in the male luer body 40 in order to prevent rotation of the seal 170. In addition, an axially extending, annular flange or ring 175 is provided on the distal face 176 of the seal 170. The flange 175 fits within the groove 49 in the receptacle 40 of the male luer body 10 in the manner of an O-ring, in order to maintain a seal at higher pressures. The flange 175 also allows the seal 170 to maintain a seal when fluid flow in either direction through the present connector assembly 1 , and also helps to prevent fluid from passing around the seal 170 rather than through the flow passageway 171.

### Connector Assembly

To assemble the present connector assembly 1, the stopper 160 is placed around the channel insert 150, and this subassembly is placed through the distal end 104 of the female luer body 100 to form a female luer subassembly. The seal 170 is then placed in the distal end 44 of the receptacle 40 of the male luer body 10 such that the flange 175 is seated within the groove 49 in the receptacle 40 and the seal pegs 41 are retained by the grooves 173, thereby forming a male luer subassembly. The female luer subassembly can then be joined to the male luer subassembly by placing the distal end 104 of the female luer body 100 within the proximal end 12 of the male luer body 10 such that the cam post 110 on the female luer body 100 is placed within the cam groove 50 of the male luer body 10. The female luer body 100 can then be rotated in order to secure the subassemblies to one another.

Except as described otherwise herein with respect to the stopper 160 and seal 170, the components of the connector assembly 1 can be made of any suitably rigid material used for medical applications, such as polycarbonate or other medical grade plastic.

Although the present invention has been described in considerable detail with reference to certain preferred embodiments, other embodiments are possible. Therefore, the scope of the appended claims should not be limited to the description of preferred embodiments contained in this disclosure. Recitation of value ranges herein is merely intended to serve as a shorthand method for referring individually to each separate value falling within the range. Unless otherwise indicated herein, each individual value is incorporated into the specification as if it were individually recited herein.

## Claims

1. A medical connector assembly (1) having a proximal portion (2) and a distal portion (4) and defining a longitudinal axis therebetween, said assembly comprising:
a male luer body (10) having a proximal end (12), a distal end (14), and a flow channel (25) extending between said proximal end (12) and said distal end (14) of the male luer body (10), said distal end (14) of the male luer body (10) comprising a tapered male luer member (20) having an open end (27) adapted to be connected to a fluid conduit;
said proximal end (12) of the male luer body (10) further comprising a receptacle (40) for receiving a female luer assembly (105);
said female luer assembly (105), comprising:
a female luer body (100) having a proximal end (102), a distal end (104), and a flow channel (101) extending between said proximal end (102) and said distal end (104) of said female luer body (100), said proximal end (102) of said female luer body (100) forming a female luer member for receiving a male luer member of a fluid transfer device;
a fluid channel insert (150) having a proximal end (152), a distal end (154), and a flow channel (140, 140a) extending between said proximal end (152) and distal end (154) of the fluid channel insert (150), wherein said flow channel (140, 140a) of said fluid channel insert (150) comprises a distal channel (140a) and an interior channel (140) arranged at an angle to each other, to position a channel distal opening (143) is offset from said longitudinal axis at said distal end (154) of said fluid channel insert (150), said fluid channel insert (150) further comprising a distal annular rim (142) at its distal end (154), said annular rim (142) engaging said distal end (104) of said female luer body (100), said proximal end (152) of said fluid channel insert (150) comprising a tubular conduit (153) having an opening (149) at said proximal end (152), an interior surface (155) of said flow channel (140) proximate to said opening (149) comprising longitudinally extending grooves (157), the fluid channel insert (150) having an annular barb-shaped flange (141) extending outwardly from the tubular conduit (153);
a cylindrical shaped elastomeric seal (170) having a proximal end (172), a distal end (174), and a flow channel (171) extending between said proximal end (172) and distal end (174), said flow channel extending from an off-center positioned proximal end (172) to an axially aligned distal end (174), said seal (170) being located between a distal end (154) of the channel insert (150) and a distal end (44) of the receptacle (40) of the male luer body (10);
wherein said flow channel (171) of said elastomeric seal (170) is extends from an off-center positioned opening (179) at the proximal end (172)) to an axially aligned distal end (174) said opening being adapted to provide fluid communication with said distal flow channel (140a) of said fluid channel insert (150) at said distal end (154) of said fluid channel insert (150); and
an elastomeric stopper (160) having a proximal end (162) and a distal end (164), the stopper comprising a hollow, longitudinal structure having bellows or accordion-like folds (161);
said elastomeric stopper (160) having a flush surface at its proximal end (162), said flush surface (182) comprising a slit (181) to allow an opening to be formed by deforming said proximal end (162), further comprising an annular groove (184) adapted to mate with said a annular barb-shaped flange (141) of said fluid channel insert (150),
wherein the elastomeric stopper (160) is positioned such that an interior surface (166) of the stopper surrounds the exterior surface of the tubular conduit (153) of the channel insert (150) to form a subassembly, said subassembly being placed through the distal end (104) of the female luer body (100) to form the female luer subassembly; and wherein a flow channel is formed through said medical connector assembly (1) when said male luer body, female luer body, fluid channel insert and elastomeric seal flow channels (25; 101; 140; 171) are aligned.

2. The medical connector assembly (1) of claim 1, further comprising a cap (15), said cap (15) having an open proximal end, a closed distal end, and a longitudinal axis therebetween, said proximal end being sized to fit on said male luer member of said male luer body (10).

3. The medical connector assembly (1) as set forth in claim 1, wherein an exterior surface (165) of said stopper (160) and an interior surface (103) of said female luer body (100) comprise interlocking walls (114; 187) precluding removal of said stopper (160) from said female luer body (100).

4. The medical connector assembly (1) as set forth in claim 1, where a distal end (154) of said fluid channel insert (150) comprises at least one longitudinal groove for grasping said distal end (164) of said stopper (160) and hinder relative rotational movement therebetween when the medical connector assembly (1) is accessed by the male luer member of a fluid transfer device.

5. The medical connector assembly (1) as set forth in claim 4, comprising a plurality of said longitudinal grooves of said fluid channel insert (150) oriented frustroconically.

6. The medical connector assembly (1) as set forth in claim 1, wherein said distal portion of said female luer body (100) and said distal portion of said fluid channel insert (150) comprise interlocking grooves and projections (159; 138) precluding relative rotation between said female luer body (100) and said fluid channel insert (150).

7. The medical connector assembly (1) as set forth in claim 6, wherein said interlocking grooves (159) are formed on an outside surface (146) of said fluid channel insert (150) and said interlocking projections (138) are formed on an inside surface (136) of said female luer body (100).

8. The medical connector assembly (1) as set forth in claim 7, wherein said interlocking grooves and projections (159, 138) are oriented frustroconically.

9. The medical connector assembly (1) as set forth in claim 1, wherein said female luer body (100) and said male luer body (10) comprise an interlocking cam groove and interlocking cam post (50;110) that preclude over-rotation of said female body (100) relative to said male luer body (10).

10. The medical connector assembly (1) as set forth in claim 9, wherein said male luer body (10) includes said interlocking cam groove (50) and said female luer body (100) includes said interlocking cam post (110).

## Patentansprüche

1. Medizinische Anschlussanordnung (1), die einen proximalen Abschnitt (2) und einen distalen Abschnitt (4) aufweist und eine Längsachse dazwischen definiert, wobei die Anordnung umfasst:
einen männlichen Luer-Körper (10) mit einem proximalen Ende (12), einem distalen Ende (14) und einem Fließkanal (25), der sich zwischen dem proximalen Ende (12) und dem distalen Ende (14) des männlichen Luer-Körpers (10) erstreckt, wobei das distale Ende (14) des männlichen Luer-Körpers (10) ein konisches männliches Luer-Element (20) mit einem offenen Ende (27) umfasst, das dazu angepasst ist, mit einem Fluidkanal verbunden zu werden;
wobei das proximale Ende (12) des männlichen Luer-Körpers (10) ferner eine Aufnahme (40) zum Aufnehmen einer weiblichen Luer-Anordnung (105) umfasst;
wobei die weibliche Luer-Anordnung (105) umfasst:
einen weiblichen Luer-Körper (100) mit einem proximalen Ende (102), einem distalen Ende (104) und einem Fließkanal (101), der sich zwischen dem proximalen Ende (102) und dem distalen Ende (104) des weiblichen Luer-Körpers (100) erstreckt, wobei das proximale Ende (102) des weiblichen Luer-Körpers (100) ein weibliches Luer-Element zum Aufnehmen eines männlichen Luer-Elements einer Fluidtransfereinrichtung bildet;
einen Fluidkanaleinsatz (150) mit einem proximalen Ende (152), einem distalen Ende (154) und einem Fließkanal (140, 140a), der sich zwischen dem proximalen Ende (152) und dem distalen Ende (154) des Fluidkanaleinsatzes (150) erstreckt, wobei der Fließkanal (140, 140a) des Fließkanaleinsatzes (150) einen distalen Kanal (140a) und einen Innenkanal (140) umfasst, die in einem Winkel zu einander angeordnet sind, um eine distale Kanalöffnung (143) verschoben von der Längsachse an dem distalen Ende (154) des Fluidkanaleinsatzes (150) zu positionieren, wobei der Fluidkanaleinsatz (150) ferner einen distalen ringförmigen Rand (142) an seinem distalen Ende (154) umfasst, wobei der ringförmige Rand (142) das distale Ende (104) des weiblichen Luer-Körpers (100) greift, wobei das proximale Ende (152) des Kanaleinsatzes (150) eine rohrförmige Leitung (153) mit einer Öffnung (149) an dem proximalen Ende (152), einer Innenfläche (155) des Fließkanals (140) nahe der Öffnung (149) umfasst, die sich in Längsrichtung erstreckende Nuten (157) umfasst, wobei der Fluidkanaleinsatz (150) einen ringförmigen widerhakenförmigen Flansch (141) aufweist, der sich von der rohrförmigen Leitung (153) nach außen erstreckt;
eine zylindrisch geformte Elastomerdichtung (170), die ein proximales Ende (172), ein distales Ende (174) und einen Fließkanal (171) aufweist, der sich zwischen dem proximalen Ende (172) und dem distalen Ende (174) erstreckt, wobei sich der Fließkanal von einem außermittig positionierten proximalen Ende (172) zu einem axial ausgerichteten distalen Ende (174) erstreckt, wobei sich die Dichtung (170) zwischen einem distalen Ende (154) des Kanaleinsatzes (150) und einem distalen Ende (44) der Aufnahme (40) des männlichen Luer-Körpers (10) befindet;
wobei sich der Fließkanal (171) der Elastomerdichtung (170) von einer außermittig positionierten Öffnung (179) an dem proximalen Ende (172) bis zu einem axial ausgerichteten distalen Ende (174) erstreckt, wobei die Öffnung dazu angepasst ist, Fluidverbindung mit dem distalen Fließkanal (140a) des Fluidkanaleinsatzes (150) an dem distalen Ende (154) des Fluidkanaleinsatzes (150) bereitzustellen; und
einen Elastomerstopfen (160), der ein proximales Ende (162) und ein distales Ende (164) aufweist, wobei der Stopfen eine hohle Längsstruktur mit Faltenbälgen oder Akkordionfaltungen (161) umfasst;
wobei der Elastomerstopfen (160) eine ebene Oberfläche an seinem proximalen Ende (162) aufweist, wobei die ebene Oberfläche (182) einen Schlitz (181) umfasst, um zu ermöglichen, dass durch Verformen des proximalen Endes (162) eine Öffnung gebildet werden kann, ferner umfassend eine ringförmige Nut (184), die dazu angepasst ist, sich mit dem ringförmigen widerhakenförmigen Flansch (141) des Fluidkanaleinsatzes (150) zu verbinden,
wobei der Elastomerstopfen (160) so positioniert ist, dass eine Innenfläche (166) des Stopfens die Außenfläche der Rohrleitung (153) des Kanaleinsatzes (150) umgibt, um eine Unteranordnung zu bilden, wobei die Unteranordnung durch das distale Ende (104) des weiblichen Luer-Körpers (100) platziert wird, um die weibliche Luer-Unteranordnung zu bilden; und wobei ein Fließkanal durch die medizinische Anschluss-Anordnung (1) gebildet ist, wenn der männliche Luer-Körper, der weibliche Luer-Körper, der Fluidkanaleinsatz und die Elastomerdichtungsfließkanäle (25; 101; 140; 171) ausgerichtet werden.

2. Medizinische Anschlussanordnung (1) nach Anspruch 1, ferner umfassend einen Aufsatz (15), wobei der Aufsatz (15) ein offenes proximales Ende, ein geschlossenes distales Ende und eine Längsachse dazwischen aufweist, wobei das proximale Ende so bemessen ist, dass es auf das männliche Luer-Element des männlichen Luer-Körpers (10) passt.

3. Medizinische Anschlussanordnung (1) nach Anspruch 1, wobei eine Außenfläche (165) des Stopfens (160) und eine Innenfläche (103) des weiblichen Luer-Körpers (100) ineinandergreifende Wände (114, 187) umfassen, die die Entfernung des Stopfens (160) aus dem weiblichen Luer-Körper (100) verhindern.

4. Medizinische Anschlussanordnung (1) nach Anspruch 1, wobei ein distales Ende (154) des Fluidkanaleinsatzes (150) mindestens eine Längsnut zum Greifen des distalen Endes (164) des Stopfens (160) umfasst und die relative Drehbewegung dazwischen verhindert, wenn von dem männlichen Luer-Element einer Fluidtransfereinrichtung auf die medizinische Anschlussanordnung (1) zugegriffen wird.

5. Medizinische Anschlussanordnung (1) nach Anspruch 4, umfassend eine Vielzahl von Längsnuten des Fluidkanaleinsatzes (150), die frustokonisch ausgerichtet sind.

6. Medizinische Anschlussanordnung (1) nach Anspruch 1, wobei der distale Abschnitt des weiblichen Luer-Körpers (100) und der distale Abschnitt des Fluidkanaleinsatzes (150) ineinandergreifende Nuten und Vorsprünge (159; 138) umfassen, die die relative Drehung zwischen dem weiblichen Luer-Körper (100) und dem Fluidkanaleinsatz (150) verhindern.

7. Medizinische Anschlussanordnung (1) nach Anspruch 6, wobei die ineinandergreifenden Nuten (159) an einer Außenseite (146) des Fluidkanaleinsatzes (150) gebildet sind, und die ineinandergreifenden Vorsprünge (138) an einer Innenseite (136) des weiblichen Luer-Körpers (100) gebildet sind.

8. Medizinische Anschlussanordnung (1) nach Anspruch 7, wobei die ineinandergreifenden Nuten und Vorsprünge (159, 138) frustokonisch ausgerichtet sind.

9. Medizinische Anschlussanordnung (1) nach Anspruch 1, wobei der weibliche Luer-Körper (100) und der männliche Luer-Körper (10) eine ineinandergreifende Nockennut und einen ineinandergreifenden Nockenstab (50, 110) umfassen, die eine Überdrehung des weiblichen Körpers (100) bezogen auf den männlichen Luer-Körper (10) verhindern.

10. Medizinische Anschlussanordnung (1) nach Anspruch 9, wobei der männliche Luer-Körper (10) die ineinandergreifende Nut (50) umfasst und der weibliche Luer-Körper (100) den ineinandergreifenden Nockenstab (110) umfasst.

## Revendications

1. Ensemble raccord médical (1) ayant une partie proximale (2) et une partie distale (4) et définissant un axe longitudinal entre elles, ledit ensemble comprenant :
un corps Luer mâle (10) ayant une extrémité proximale (12), une extrémité distale (14), et un canal d'écoulement (25) s'étendant entre ladite extrémité proximale (12) et ladite extrémité distale (14) du corps Luer mâle (10), ladite extrémité distale (14) du corps Luer mâle (10) comprenant un élément Luer mâle conique (20) ayant une extrémité ouverte (27) adaptée pour être raccordée à une conduite de fluide ;
ladite extrémité proximale (12) du corps Luer mâle (10) comprenant en outre un réceptacle (40) destiné à recevoir un ensemble Luer femelle (105) ;
ledit ensemble Luer femelle (105) comprenant ;
un corps Luer femelle (100) ayant une extrémité proximale (102), une extrémité distale (104), et un canal d'écoulement (101) s'étendant entre ladite extrémité proximale (102) et ladite extrémité distale (104) dudit corps Luer femelle (100), ladite extrémité proximale (102) et dudit corps Luer femelle (100) formant un élément Luer femelle destiné à recevoir un élément Luer mâle d'un dispositif de transfert de fluide ;
un canal de fluide rapporté (150) ayant une extrémité proximale (152), une extrémité distale (154), et un canal d'écoulement (140, 140a) s'étendant entre lesdites extrémité proximale (152) et extrémité distale (154) du canal de fluide rapporté (150), ledit canal d'écoulement (140, 140a) dudit canal de fluide rapporté (150) comprenant un canal distal (140a) et un canal intérieur (140) disposés à un angle l'un par rapport à l'autre, pour positionner une ouverture distale de canal (143) en décalage par rapport audit axe longitudinal à ladite extrémité distale (154) dudit canal de fluide rapporté (150), ledit canal de fluide rapporté (150) comprenant en outre un bord annulaire distal (142) à son extrémité distale (154), ledit bord annulaire (142) s'enclenchant avec ladite extrémité distale (104) dudit corps Luer femelle (100), ladite extrémité proximale (152) dudit canal de fluide rapporté (150) comprenant une conduite tubulaire (153) ayant une ouverture (149) à ladite extrémité proximale (152), une surface intérieure (155) dudit canal d'écoulement (140) à proximité de ladite ouverture (149) comprenant des rainures s'étendant longitudinalement (157), le canal de fluide rapporté (150) ayant une collerette annulaire en forme d'ardillon (141) s'étendant vers l'extérieur depuis la conduite tubulaire (153) ;
un joint d'étanchéité élastomère de forme cylindrique (170) ayant une extrémité proximale (172), une extrémité distale (174), et un canal d'écoulement (171) s'étendant entre lesdites extrémité proximale (172) et extrémité distale (174), ledit canal d'écoulement s'étendant depuis une extrémité proximale positionnée en décalage par rapport au centre (172) jusqu'à une extrémité distale alignée axialement (174), ledit joint d'étanchéité (170) étant situé entre une extrémité distale (154) du canal rapporté (150) et une extrémité distale (44) du réceptacle (40) du corps Luer mâle (10) ;
ledit canal d'écoulement (171) dudit joint d'étanchéité élastomère (170) s'étendant depuis une ouverture positionnée en décalage par rapport au centre (179) à l'extrémité proximale (172) jusqu'à une extrémité distale alignée axialement (174), ladite ouverture étant adaptée pour fournir une communication fluidique avec ledit canal d'écoulement distal (140a) dudit canal de fluide rapporté (150) à ladite extrémité distale (154) dudit canal de fluide rapporté (150) ; et
une butée élastomère (160) ayant une extrémité proximale (162) et une extrémité distale (164), la butée comprenant une structure creuse, longitudinale ayant des soufflets ou des plis en forme d'accordéon (161) ;
ladite butée élastomère (160) ayant une surface affleurante à son extrémité proximale (162), ladite surface affleurante (182) comprenant une fente (181) pour permettre de former une ouverture en déformant ladite extrémité proximale (162), comprenant en outre une rainure annulaire (184) adaptée pour s'accoupler avec ladite collerette annulaire en forme d'ardillon (141) dudit canal de fluide rapporté (150),
la butée élastomère (160) étant positionnée de telle sorte qu'une surface intérieure (166) de la butée entoure la surface extérieure de la conduite tubulaire (153) du canal rapporté (150) pour former un sous-ensemble, ledit sous-ensemble étant placé à travers l'extrémité distale (104) du corps Luer femelle (100) pour former le sous-ensemble Luer femelle ; et un canal d'écoulement étant formé à travers ledit ensemble raccord médical (1) quand lesdits corps Luer mâle, corps Luer femelle, canal de fluide rapporté et canaux d'écoulement de joint d'étanchéité élastomère (25 ; 101 ; 140 ; 171) sont alignés.

2. Ensemble raccord médical (1) de la revendication 1, comprenant en outre un embout (15), ledit embout (15) ayant une extrémité proximale ouverte, une extrémité distale fermée, et un axe longitudinal entre elles, ladite extrémité proximale étant dimensionnée pour s'ajuster sur ledit élément Luer mâle dudit corps Luer mâle (10).

3. Ensemble raccord médical (1) tel que défini dans la revendication 1, dans lequel une surface extérieure (165) de ladite butée (160) et une surface intérieure (103) dudit corps Luer femelle (100) comprennent des parois à emboîtement (114 ; 187) empêchant le retrait de ladite butée (160) dudit corps Luer femelle (100).

4. Ensemble raccord médical (1) tel que défini dans la revendication 1, dans lequel une extrémité distale (154) dudit canal de fluide rapporté (150) comprend au moins une rainure longitudinale destinée à entrer en prise avec ladite extrémité distale (164) de ladite butée (160) et faire obstacle à un mouvement de rotation relatif entre elles quand l'élément Luer mâle d'un dispositif de transfert de fluide accède à l'ensemble raccord médical (1).

5. Ensemble raccord médical (1) tel que défini dans la revendication 4, comprenant une pluralité desdites rainures longitudinales dudit canal de fluide rapporté (150) orientées de façon tronconique.

6. Ensemble raccord médical (1) tel que défini dans la revendication 1, dans lequel ladite partie distale dudit corps Luer femelle (100) et ladite partie distale dudit canal de fluide rapporté (150) comprennent des rainures et saillies à emboîtement (159 ; 138) empêchant une rotation relative entre ledit corps Luer femelle (100) et ledit canal de fluide rapporté (150).

7. Ensemble raccord médical (1) tel que défini dans la revendication 6, dans lequel lesdites rainures à emboîtement (159) sont formées sur une surface extérieure (146) dudit canal de fluide rapporté (150) et lesdites saillies à emboîtement (138) sont formées sur une surface intérieure (136) dudit corps Luer femelle (100).

8. Ensemble raccord médical (1) tel que défini dans la revendication 7, dans lequel lesdites rainures et saillies à emboîtement (159, 138) sont orientées de façon tronconique.

9. Ensemble raccord médical (1) tel que défini dans la revendication 1, dans lequel ledit corps Luer femelle (100) et ledit corps Luer mâle (10) comprennent une rainure de came à emboîtement et un tenon de came à emboîtement (50 ; 110) qui empêchent une rotation excessive dudit corps femelle (100) par rapport audit corps Luer mâle (10).

10. Ensemble raccord médical (1) tel que défini dans la revendication 9, dans lequel ledit corps Luer mâle (10) comporte ladite rainure de came à emboîtement (50) et ledit corps Luer femelle (100) comporte ledit tenon de came à emboîtement (110).
